Europäisches Patentamt

European Patent Office

Office européen des brevets

(11) Numéro de publication : **0 470 903 A1**

# (12) DEMANDE DE BREVET EUROPEEN

(21) Numéro de dépôt : **91402191.0**

(22) Date de dépôt : **06.08.91**

(51) Int. Cl.$^5$ : **A61B 17/32**

(30) Priorité : **10.08.90 FR 9010268**

(43) Date de publication de la demande :
**12.02.92 Bulletin 92/07**

(84) Etats contractants désignés :
**AT BE CH DE DK ES FR GB GR IT LI LU NL SE**

(71) Demandeur : **CENDIS MEDICAL SARL**
**6, Avenue de la Porte de Montrouge**
**F-75014 Paris (FR)**

(72) Inventeur : **Thein, Rafael**
**15 Moskovich St.**
**Rehovot 76474 (IL)**

(74) Mandataire : **Hasenrader, Hubert et al**
**Cabinet BEAU DE LOMENIE 55, rue**
**d'Amsterdam**
**F-75008 Paris (FR)**

(54) **Appareil pour le prélèvement d'un tendon rotulien.**

(57)   La présente invention concerne un appareil pour le prélèvement d'un tendon rotulien avec ses points d'attache osseux comprenant une première partie (1) formant moyen de préhension et une seconde partie (2) formant moyen de découpe desdits points d'attache caractérisé en ce que ladite seconde partie est constituée d'une plaque (3) formant zone de frappe et sur les bords latéraux de laquelle sont montées deux lames verticales parallèles (4, 4') dont le bord inférieur est tranchant.

FIG.1

EP 0 470 903 A1

La présente invention concerne un appareil pour le prélèvement d'un tendon rotulien.

Les tendons rotuliens comportent des points d'attache osseux qu'il faut prélever également avec le reste du tendon afin de faciliter la greffe ultérieure.

Il est donc nécessaire d'effectuer une découpe symétrique de l'os rotulien autour du point d'attache du tendon.

La présente invention a pour but de réaliser cette opération de manière satisfaisante.

Ce but est atteint conformément à l'invention par un appareil pour le prélèvement d'un tendon rotulien avec ses points d'attache osseux comprenant une première partie formant moyen de préhension et une seconde partie formant moyen de découpe desdits points d'attache caractérisé en ce que ladite seconde partie est constituée d'une plaque formant zone de frappe et sur les bords latéraux de laquelle sont montées deux lames verticales parallèles, dont le bord inférieur est tranchant.

Selon une autre caractéristique de l'invention, lesdites lames verticales sont amovibles ; ladite plaque horizontale étant pourvue sur les bords latéraux de sa face inférieure de rainures de guidage et de retenue destinées à recevoir de façon coulissante le bord supérieur des lames verticales.

Selon encore une autre caractéristique, ladite plaque horizontale est pourvue à au moins l'une de ses extrémités longitudinales d'une fente transversale, dont la longueur est sensiblement égale à la largeur de ladite plaque et dont la largeur est suffisante pour pouvoir y insérer la lame verticale d'un ciseau à frapper.

L'invention sera mieux comprise à la lecture de la description qui va suivre accompagnée des dessins annexés sur lesquels :

La figure 1 représente une vue en perspective de l'appareil de l'invention.

La figure 2 représente une variante de réalisation de l'appareil de la figure 1.

La figure 3 représente une vue de détail en perspective d'un mode de fixation des lames en position démontée.

La figure 4 représente le mode de fixation de la figure 3 avec les lames en position de verrouillage.

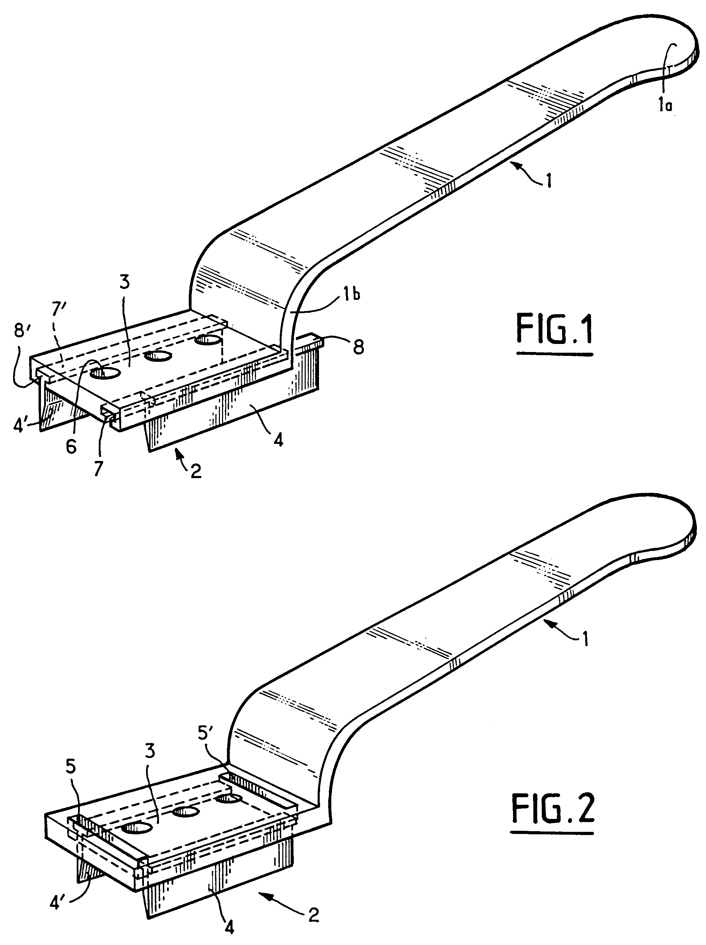

L'appareil tel que représenté sur la figure 1 comprend une première partie 1 formant moyen de préhension pour l'utilisateur et une seconde partie 2 formant moyen de découpe du point d'attache osseux du tendon.

La première partie 1 est constituée d'un profilé plat s'étendant sur la plus grande partie de sa longueur de façon inclinée par rapport au plan de ladite seconde partie 2 et perpendiculairement à ladite plaque au niveau de sa zone de liaison 1b avec elle.

Selon un autre mode de réalisation, les première et seconde parties sont d'une seule pièce d'une extrémité à l'autre.

De manière avantageuse, l'extrémité libre 1a de la première partie 1 a un profil recourbé et/ou de section transversale sensiblement en auge en formant une spatule dont la largeur est de préférence inférieure ou égale à 10 mm.

La seconde partie 2 est constituée d'une plaque rectangulaire 3 formant zone de frappe pour un outil lourd afin de réaliser la découpe de la zone osseuse entourant le point d'attache du tendon.

Des lames verticales parallèles 4, 4' sont montées sur les bords latéraux de la plaque 3.

Les lames 4, 4' possèdent un bord inférieur suffisamment tranchant pour découper la zone osseuse sous l'action d'un outil de frappe sur la face supérieure de la plaque 3.

Le bord inférieur sera biseauté sur une seule face ou les deux selon les cas.

L'écartement entre les lames 4, 4' au niveau de leur bord inférieur tranchant dépend du profil de ce bord et du montage des lames sur la plaque 3.

Dans le cas où le bord inférieur des lames 4, 4' n'est biseauté que sur une seule face, l'écartement maximum est obtenu lorsque les deux lames sont montées sur la plaque 3 avec leur face biseautée en regard.

D'une manière avantageuse, l'écartement entre les lames sera compris entre 6 et 14 mm.

Dans un mode de réalisation avantageux, la plaque 3 est pourvue, au moins à son extrémité longitudinale avant d'une fente transversale 5 dont la longueur correspond sensiblement à la largeur de la plaque 3 et dont la longueur est suffisante pour pouvoir y insérer la lame verticale d'un ciseau à frapper destiné à effectuer la découpe avant de la zone osseuse du point d'attache.

La fente 5 permet de positionner correctement la lame du ciseau et de réaliser la découpe avant de manière jointive par rapport aux découpes latérales effectuées au moyen des lames parallèles 4.

Sur la figure 2, la plaque 3 est également pourvue d'une fente transversale arrière 5' réalisée au niveau de la zone de liaison entre la partie 1 et la plaque 3.

En effet, après mise en place de la partie tranchante 2 de l'appareil sur le point d'attache du tendon sur le tibia et découpe de la zone osseuse environnante, l'opérateur déplace l'appareil au-dessus et le long du tendon jusqu'à la rotule en l'égalisant latéralement pour lui donner une largeur déterminée, égale à la distance entre les lames 4, 4'.

Cette égalisation de la largeur du tendon permet d'obtenir un organe biologique qui fera fonction de ligament une fois greffé. Une fois amenée au contact de la rotule, la fente arrière 5' est donc située en bout du point d'attache rotulien.

C'est donc au moyen de cette fente 5' que l'opérateur pourra éventuellement repérer par entaillage sur l'os rotulien la ligne de découpe transversale.

Il fait de même par appui sur la plaque 3 pour que les lames 4, 4' entaillent superficiellement l'os rotulien qui est relativement fragile et qui sera donc découpé, autour du point d'attache par d'autres moyens.

Les fentes 5, 5' peuvent également être réalisées avec des parois internes avant et arrière inclinées par rapport à la verticale d'un angle inférieur à 30° pour permettre une découpe osseuse de biais.

La plaque 3 est aussi pourvue d'orifices 6 disposés selon un axe sensiblement longitudinal et destinés au passage d'un outil de perçage tel qu'une mèche.

De manière avantageuse, il est prévu de réaliser des zones surélevées sur la plaque 3 formant enclumes et destinées à protéger les orifices 6 ainsi que les moyens de fixation latéraux des lames amovibles 4, 4', lors de la frappe sur la plaque 3.

De manière avantageuse, les lames 4, 4' sont amovibles étant engagées par leur bord supérieur 8, 8' de manière coulissante dans des moyens de fixation constitués de rainures de guidage et de retenue 7, 7' réalisées sur les bords latéraux de la face inférieure de la plaque 3.

A cet effet, le bord supérieur 8, 8' est réalisé sous la forme d'un profilé dont la section verticale est également en T et dont les dimensions sont déterminées en fonction des dimensions de la rainure 7, 7' pour y coulisser librement.

Les rainures 7, 7' peuvent être sous forme de T ou de queue d'aronde afin de permettre une mise en place ou un remplacement aisé et rapide des lames 4, 4' ainsi qu'une bonne tenue de ces dernières sur la plaque 3.

Le bord supérieur des lames 4, 4' est glissé dans les rainures 7, 7' par l'arrière de la plaque 3 et vient en butée à l'avant de ladite plaque.

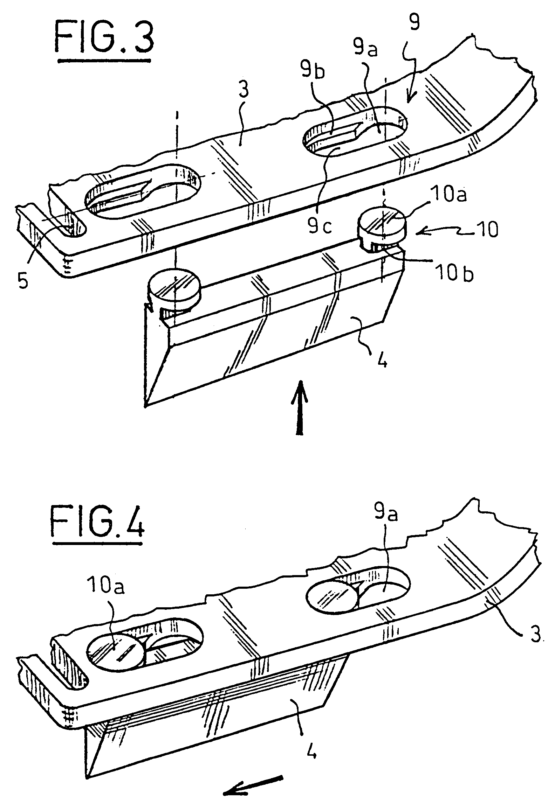

Selon un autre mode de réalisation illustré par les figures 3 et 4, les moyens de fixation des lames amovibles 4, 4' comprennent des ouvertures discontinues 9 ménagées sur les bords latéraux et au travers de la plaque 3. Ces ouvertures 9 sont formées d'un conduit vertical 9a prolongé dans l'épaisseur de la plaque 3 par une gorge horizontale 9b débouchant sur la face supérieure de la plaque 3 et comportant sur le fond une fente longitudinale 9c mettant en communication la face inférieure et la face supérieure de la plaque 3.

Les lames 4, 4' sont quant à elles pourvues sur leur bord supérieur 8, 8' d'éléments d'accrochage réalisés sous la forme de plots verticaux 10 dont la tête 10a a un diamètre plus large que la tige 10b et qui sont adaptés pour venir s'engager selon un mode de fixation dit à baïonnette dans les ouvertures 9 de la plaque 3. L'opération d'accrochage s'effectue en introduisant tout d'abord les plots 10 des lames 4, 4' dans les conduits 9a puis en déplaçant ces derniers par translation longitudinale le long de la gorge 9b, la tige 10b des plots 10 étant engagée au travers de la fente 9c et la tête 10a reposant sur le fond de la gorge 9b.

Ce mode de réalisation permet l'affûtage et le remplacement des lames usées. Il permet également d'orienter les lames 4, 4' avec un bord biseauté à l'intérieur ou à l'extérieur en fonction du type de découpe à effectuer et donne ainsi à l'appareil de l'invention une efficacité et une précision de découpe remarquables.

**Revendications**

1. Appareil pour le prélèvement d'un tendon rotulien avec ses points d'attache osseux comprenant une première partie (1) formant moyen de préhension et une seconde partie (2) formant moyen de découpe desdits points d'attache caractérisé en ce que ladite seconde partie est constituée d'une plaque (3) formant zone de frappe et sur les bords latéraux de laquelle sont montées deux lames verticales parallèles (4, 4') dont le bord inférieur est tranchant.

2. Appareil selon la revendication 1, caractérisé en ce que lesdites lames verticales (4, 4') sont amovibles.

3. Appareil selon la revendication 1 ou 2, caractérisé en ce que ladite plaque est pourvue à au moins l'une de ses extrémités longitudinales d'une fente transversale (5, 5') dont la longueur est sensiblement égale à la largeur de ladite plaque (3) et dont la largeur est suffisante pour pouvoir y insérer la lame verticale d'un ciseau à frapper.

4. Appareil selon l'une des revendications précédentes caractérisé en ce que ladite plaque (3) est pourvue d'orifices (6) disposés selon un axe sensiblement longitudinal et destinés au passage de mèches.

5. Appareil selon la revendication 2, caractérisé en ce que ladite plaque est pourvue sur les bords latéraux de sa face inférieure de rainures de guidage et de retenue (7, 7') destinées à recevoir de façon coulissante le bord supérieur (8, 8') des lames verticales (4, 4').

6. Appareil selon l'une des revendications précédentes caractérisé en ce que ladite première partie (1) formant moyen de préhension est constituée d'un profilé plat s'étendant sur la plus grande partie de sa longueur de façon inclinée par rapport au plan de ladite plaque et perpendiculairement à ladite plaque au niveau de leur zone de liaison.

7. Appareil selon la revendication 6, caractérisé en ce que l'extrémité libre (la) de ladite première partie a un profil formant spatule.

8. Appareil selon l'une des revendications précédentes, caractérisé en ce que le bord inférieur desdites lames verticales (4, 4') est biseauté sur une seule face.

FIG.1

FIG.2

## FIG.3

## FIG.4

Office européen
des brevets

**RAPPORT DE RECHERCHE EUROPEENNE**

Numero de la demande

EP 91 40 2191

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-4 239 045 (SCHLEIN) <br> * revendication 1; figures 1-3 * <br> --- | 1 | A 61 B 17/32 |
| A | SOVIET INVENTIONS ILLUSTRATED section Mechanical, semaine 8403, 29 février 1984, abrégé no. 015954 P/31, Derwent Publications Ltd., London, GB; & SU - A - 1003828 (IRKUT TRAUMATHOLOGY) 23.09.1981 <br> --- | 1 | |
| A | US-A-3 221 744 (STRYKER) <br> * revendication 1; colonne 2, lignes 28-42 * <br> --- | 1 | |
| A | US-A-1 328 459 (SMITH) <br> * revendication 1; figure 1 * <br> ----- | 1 | |

DOMAINES TECHNIQUES RECHERCHES (Int. Cl.5)

A 61 B

Le présent rapport a été établi pour toutes les revendications

| Lieu de la recherche | Date d'achèvement de la recherche | Examinateur |
|---|---|---|
| BERLIN | 13-09-1991 | PAPA E.R. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

............................................................................

& : membre de la même famille, document correspondant

EPO FORM 1503 03.82 (P0402)